(19) Europäisches Patentamt
European Patent Office
Office européen des brevets

(11) Veröffentlichungsnummer: **0 126 949**
**B1**

# (12) EUROPÄISCHE PATENTSCHRIFT

(45) Veröffentlichungstag der Patentschrift: **27.09.89**

(51) Int. Cl.⁴: **C 07 D 239/26**, G 02 F 1/13

(21) Anmeldenummer: **84104341.7**

(22) Anmeldetag: **17.04.84**

(54) **Fluorhaltige Pyrimidinderivate.**

(30) Priorität: **27.04.83 DE 3315295**

(43) Veröffentlichungstag der Anmeldung: **05.12.84 Patentblatt 84/49**

(45) Bekanntmachung des Hinweises auf die Patenterteilung: **27.09.89 Patentblatt 89/39**

(84) Benannte Vertragsstaaten: **CH DE FR GB LI**

(56) Entgegenhaltungen: **EP-A- 0 025 119**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **Krause, Joachim, Dr., Samuel-Morse-Strasse 14, D-6110 Dieburg (DE)**
Erfinder: **Römer, Michael, Dr., Im Grossen Garten 18, D-6054 Rodgau 2 (DE)**
Erfinder: **Pohl, Ludwig, Dr., Niebergallweg 5, D-6100 Darmstadt (DE)**
Erfinder: **Scheuble, Bernhard, Dr., Grenzweg 18, D-6146 Alsbach-Hähnlein 1 (DE)**
Erfinder: **Weber, Georg, Wilhelm-Leuschner-Strasse 38, D-6101 Erzhausen (DE)**

EP 0 126 949 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue fluorhaltige Pyrimidinderivate, die zur Verwendung als Komponenten flüssigkristalliner Dielektrika bestimmt sind, und neue flüssigkristalline Dielektrika mit mindestens einem fluorhaltigen Pyrimidinderivat, die sich zur Verwendung in elektrooptischen Anzeigevorrichtungen, insbesondere solchen für Multiplexbetrieb eignen.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I gelöst.

Es wurde gefunden, daß fluorhaltige Pyrimidinderivate der Formel I

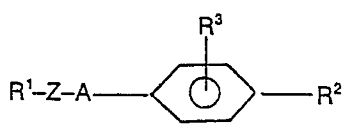
I

worin

$R^1$ und $R^2$ H, R oder OR bedeuten, wobei einer davon auch CN oder F, oder sofern A eine kovalente Bindung ist, auch 4-$R^4$-Phenyl oder 4-R-Cyclohexyl bedeuten, wobei $R^4$ R, OR, CN oder F bedeutet R eine Alkylgruppe mit 1 bis 12 C-Atomen ist,

$R^3$ F oder, wenn einer der Reste $R^1$ oder $R^2$ F oder 4-Fluorphenyl ist, auch H bedeutet,

Z 1,3-Pyrimidin-2,5-diyl und

A 1,4-Phenylen, 1,4-Cyclohexylen oder eine kovalente Bindung ist,

sowie der Formeln

| | |
|---|---|
| R–Ph–Z–PhF–CN | I dj |
| RO–Ph–Z–PhF–CN | I dk |
| R–Ph–Z–Ph–F | I dm |
| RO–Ph–Z–Ph–F | I dn |
| F–Z–Ph–Cy–R | I l |
| R–Cy–Z–Ph–F | I n |
| R–Cy–Z–PhF–F | I od |
| R–Cy–Z–PhF–CN | I oe |
| F–Z–PhF–Cy–R | I pc |
| NC–Z–PhF–Cy–R | I pd |
| R—PhF–Ph–CN | I qd |
| R–Z–PhF–Ph–F | I qe |
| NC–Z–PhF–Ph–R | I qf |
| NC–Z–PhF–Ph–OR | I qg |

worin

Ph 1,4-Phenylen,

PhF 2- bzw. 3-Fluor-1,4-phenylen,

Cy 1,4-Cyclohexylen,

R eine Alkylgruppe mit 1 bis 12 C-Atomen, und

Z 1,3-Pyrimidin-2,5-diyl ist,

hervorragend zur Herstellung von Flüssigkristallmischungen mit verringerter Tendenz zur Ausbildung smektischer Phasen bei tiefen Temperaturen geeignet sind und kleine $K_{33}/k_{11}$-Quotienten aufweisen ($k_{33}$ = elastische Biegekonstante, $k_{11}$ = elastische Spreizungskonstante; vom Quotienten $K_{33}/k_{11}$ ist u. a. die Steilheit der Kennlinie der multiplexbaren Mischung abhängig). Dabei besitzen diese Verbindungen einen breiten Anwendungsbereich: in Abhängigkeit von der Auswahl der Substituenten können sie als Basismaterialien dienen, aus denen flüssigkristalline Dielektrika zum überwiegenden Teil bestehen, es können aber auch Verbindungen der Formel I flüssigkristallinen Basismaterialien aus anderen Verbindungsklassen zugesetzt werden, um so Dielektrika mit einer verbreiterten flüssigkristallinen Mesophase herzustellen oder die Größe der dielektrischen Anisotropie eines solchen Dielektrikums zu beeinflussen.

Die Verbindungen der Formel I sind in reinem Zustand farblos und bilden niederviskose nematische Mesophasen in einem breiten und für die elektrooptische Verwendung günstig gelegenen Temperaturbereich. Chemisch, thermisch und gegen Licht sind sie sehr stabil. In der EP-A-25 119 werden ähnliche Verbindungen, nämlich kristallinflüssige 5-Alkyl-2-(4-acyloxyphenyl)-pyrimidine beschrieben.

Gegenstand der Erfindung sind somit die Verbindungen der Formel I sowie ein Verfahren zu ihrer Herstellung, dadurch gekennzeichnet, daß man

(a) ein Halogenpyrimidin der Formel II

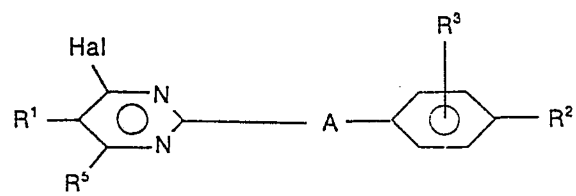
II

worin

$R^5$ H oder Hal und

Hal Cl oder Br bedeuten und

$R^1$, $R^2$, $R^3$ und A die angegebene Bedeutung haben, reduktiv dehalogeniert oder daß man

(b) eine Verbindung, die der Formel I entspricht, jedoch an Stelle eines oder mehrerer Fluoratome eine oder mehrere Aminogruppen enthält, diazotiert, in das entsprechende Diazoniumtetrafluoroborat überführt und dieses thermisch zersetzt.

Gegenstand der Erfindung sind weiterhin die Verwendung der Verbindungen der Formel I als Komponenten flüssigkristalliner Dielektrika für elektrooptische Anzeigeelemente, flüssigkristalline Dielektrika für elektrooptische Anzeigeelemente mit einem Gehalt an mindestens einer Verbindung der Formel I sowie elektrooptische Anzeigeelemente, die derartige Dielektrika enthalten.

Die erfindungsgemäßen fluorhaltigen Pyrimidinderivate der Formel I umfassen insbesondere die bevorzugten Verbindungen der nachstehenden Formeln I a bis I q

| | |
|---|---|
| $R^1$–Z–Ph–F | I a |
| $R^1$–Z–PhF–$R^2$ | I b |
| $R^1$–Z–Ph–Ph–F | I c |
| $R^4$–Ph–Z–Ph$R^3$–$R^2$ | I d |
| F–Z–Ph–$R^2$ | I e |

| | |
|---|---|
| $R^1$–Z–Ph–PhF–$R^2$ | I f |
| F–Ph–Z–Ph–Ph–$R^4$ | I g |
| F–Z–Ph–Ph–$R^2$ | I h |
| $R^1$–Z–Cy–Ph–F | I i |
| $R^1$–Z–Cy–PhF–$R^2$ | I j |
| $R^1$–Z–PhF–Cy–R | I k |
| F–Z–Ph–Cy–R | I l |
| F–Z–Cy–Ph–$R^2$ | I m |
| R–Cy–Z–Ph–F | I n |
| R–Cy–Z–PhF–$R^2$ | I o |
| $R^1$–Z–Ph$R^3$–Cy–R | I p |
| $R^1$–Z–Ph$R^3$–Ph–$R^4$ | I q |

wobei pH 1,4-Phenylen, PhF 2- bzw. 3-Fluor-1,4-phenylen und Cy 1,4-Cyclohexylen bedeutet und $R^1$, $R^2$, $R^3$ und Z die bei Formel I angegebene Bedeutung haben.

Unter den Verbindungen der vorstehenden Formeln I a bis I q sind diejenigen der Formeln I a, I c, I e, I g, In und I p, insbesondere I a, I j, I n und I p, besonders bevorzugt.

In den vor- und nachstehenden, eine 1,4-Cyclohexylengruppe enthaltenden Verbindungen der Formel I sind diejenigen bevorzugt, die eine trans-1,4-Cyclohexylengruppe enthalten.

In den Verbindungen der Formel I können die Reste $R^1$ und $R^2$ H, R oder OR, einer davon auch CN oder F bedeuten, wobei der Alkylrest R vorzugsweise geradkettig, aber auch verzweigt sein kann und bevorzugt Methyl, Ethyl, n-Propyl, n-Butyl, n-Pentyl, n-Hexyl, n-Heptyl, n-Octyl, n-Nonyl, n-Decyl, n-Undecyl oder n-Dodecyl bedeutet.

In bevorzugten Verbindungen der Formel I ist einer der Reste $R^1$ und $R^2$ Alkyl und der andere CN oder F, insbesondere CN.

Verbindungen der Formel I mit einer verzweigten Flügelgruppe $R^1$ oder $R^2$ sind gelegentlich wegen einer besseren Löslichkeit in den üblichen flüssigkristallinen Basismaterialien von Bedeutung, insbesondere aber als chirale Dotierstoffe, wenn sie durch die Kettenverzweigung optische Aktivität besitzen. Solche verzweigten Flügelgruppen enthalten bevorzugt nicht mehr als eine Kettenverzweigung. Bevorzugte verzweigte Kohlenwasserstoffreste sind die, in denen sich an einer längeren Kohlenstoffkette in 1-, 2- oder 3-Stellung eine Methyl- oder Ethylgruppe befindet, beispielsweise 2-Methylpropyl, 2-Methylbutyl, 3-Methylbutyl, 2-Methylpentyl, 3-Methylpentyl, 2-Ethylhexyl oder 1-Methylhexyl.

Diejenigen Verbindungen der Formel I sind bevorzugt, in denen $R^1$ und $R^2$ zusammen 3 bis 14, insbesondere 4 bis 12 Kohlenstoffatome enthalten. Bevorzugt sind die Verbindungen der Formel I, deren Reste $R^1$ und/oder $R^2$ Alkylgruppen R mit 2 bis 8, insbesondere 3 bis 6 Kohlenstoffatomen enthalten. Weiterhin bevorzugt sind Verbindungen der Formel I in denen einer der Reste $R^1$ oder $R^2$ 4-R-Phenyl, 4-RO-Phenyl, 4-Cyanophenyl, 4-Fluorphenyl oder trans-4-R-Cyclohexyl, insbesondere 4-Cyanophenyl oder 4-Fluorphenyl, ist.

Die bevorzugten Verbindungen der Teilformel Ia umfassen solche der Teilformeln I aa und I ab:

| | |
|---|---|
| R–Z–Ph–F | I aa |
| RO–Z–Ph–F | I ab |

Die bevorzugten Verbindungen der Teilformel I b umfassen solche der Teilformeln I ba bis I bg:

| | |
|---|---|
| R–Z–PhF–R | I ba |
| R–Z–PhF–OR | I bb |
| R–Z–PhF–CN | I bc |
| R–Z–PhF–F | I bd |
| NC–Z–PhF–R | I bf |
| NC–Z–PhF–OR | I bg |

Darunter sind diejenigen der Teilformeln I bc, I bd, I bf und I bg besonders bevorzugt.

Die bevorzugten Verbindungen der Teilformel I c umfassen solche der Teilformeln I ca und I cb:

| | |
|---|---|
| R–Z–Ph–Ph–F | I ca |
| RO–Z–Ph–Ph–F | I cb |

Die bevorzugten Verbindungen der Teilformel I d umfassen solche der Teilformeln I da bis I dq:

| | |
|---|---|
| R–Ph–Z–PhF–R | I da |
| R–Ph–Z–PhF–OR | I db |
| RO–Ph–Z–PhF–R | I dd |
| RO–Ph–Z–PhF–OR | I de |
| R–Ph–Z–PhF–CN | I dj |
| RO–Ph–Z–PhF–CN | I dk |
| R–Ph–Z–Ph–F | I dm |
| RO–Ph–Z–Ph–F | I dn |
| F–Ph–Z–Ph–R | I dp |
| F–Ph–Z–Ph–OR | I dq |

Darunter sind diejenigen der Teilformeln I dj bis I dq besonders bevorzugt.

Die bevorzugten Verbindungen der Teilformel I e umfassen solche der Teilformeln I ea und I eb:

| | |
|---|---|
| F–Z–Ph–R | I ea |
| F–Z–Ph–OR | I eb |

Die bevorzugten Verbindungen der Teilformel I f umfassen solche der Teilformeln I fa bis I fg:

| | |
|---|---|
| R–Z–Ph–PhF–R | I fa |
| R–Z–Ph–PhF–OR | I fb |
| R–Z–Ph–PhF–CN | I fd |
| R–Z–Ph–PhF–F | I fe |
| NC–Z–Ph–PhF–R | I ff |
| NC–Z–Ph–PhF–OR | I fg |

Darunter sind diejenigen der Teilformeln I fd bis I fg besonders bevorzugt.

Die bevorzugten Verbindungen der Teilformel I g umfassen solche der Teilformeln I ga und I gb:

| | |
|---|---|
| F–Ph–Z–Ph–Ph–R | I ga |
| F–Ph–Z–Ph–Ph–OR | I gb |

Die bevorzugten Verbindungen der Teilformel I h umfassen solche der Teilformeln I ha und I hb:

| | |
|---|---|
| F–Z–Ph–Ph–R | I ha |
| F–Z–Ph–Ph–OR | I hb |

Die bevorzugten Verbindungen der Teilformel I i umfassen solche der Teilformeln I ia und I ib:

| | |
|---|---|
| R–Z–Cy–Ph–F | I ia |
| RO–Z–Cy–Ph–F | I ib |

Die bevorzugten Verbindungen der Teilformel I j umfassen solche der Teilformeln I ja bis I jg:

| | |
|---|---|
| R–Z–Cy–PhF–R | I ja |
| R–Z–Cy–PhF–OR | I jb |
| R–Z–Cy–PhF–CN | I jd |
| R–Z–Cy–PhF–F | I je |
| NC–Z–Cy–PhF–R | I jf |
| NC–Z–Cy–PhF–OR | I jg |

Die bevorzugten Verbindungen der Teilformel I o umfassen solche der Teilformeln I oa bis I oe:

R–Cy–Z–PhF–R I oa
R–Cy–Z–PhF–OR I ob
R–Cy–Z–PhF–F I od
R–Cy–Z–PhF–CN I oe

Darunter sind diejenigen der Teilformeln I od und I oe besonders bevorzugt.

Die bevorzugten Verbindungen der Teilformel I p umfassen solche der Teilformeln I pa bis I pd:

R–Z–PhF–Cy–R I pa
F–Z–Ph–Cy–R I pb
F–Z–PhF–Cy–R I pc
NC–Z–PhF–Cy–R I pd

Darunter sind diejenigen der Teilformeln I pa und I pd besonders bevorzugt.

Die bevorzugten Verbindungen der Teilformel I q umfassen solche der Teilformeln I qa bis I qg:

R–Z–PhF–Ph–R I qa
R–Z–PhF–Ph–OR I qb
R–Z–PhF–Ph–CN I qd
R–Z–PhF–Ph–F I qe
NC–Z–PhF–Ph–R I qf
NC–Z–PhF–Ph–OR I qg

Darunter sind diejenigen der Teilformeln I qd bis I qg besonders bevorzugt.

Die Verbindungen der Formel I werden vorzugsweise durch reduktive Dehalogenierung der Verbindungen der Formel II hergestellt. Verbindungen der Formel II werden zweckmäßig hergestellt durch Kondensation eines Amidins der Formel III

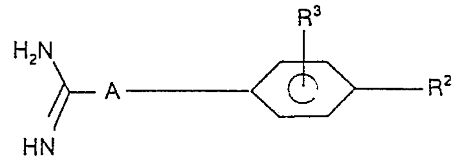

III

worin $R^2$, $R^3$ und A die angegebene Bedeutung haben, oder eines seiner funktionellen Derivate mit einem Malonsäurederivat der Formel IV, einen Formylessigsäurederivat der Formel V, einem Cyanessigsäurederivat der Formel VI oder einem Malonsäuredinitril der Formel VII,

| | | | |
|---|---|---|---|
| $R^1–CH(COOR)_2$ | $R^1–CH(CHO)(COOR)$ | $R^1–CH(CN)(COOR)$ | $R^1–CH(CN)_2$ |
| IV | V | VI | VII |

worin die Reste $R^1$ und R jeweils die angegebene Bedeutung haben, gegebenenfalls Hydrolyse und anschließende Halogenierung.

Die reduktive Dehalogenierung der Verbindungen der Formel II gelingt nach an sich bekannten Methoden, z. B. zweckmäßig mit Wasserstoff in Gegenwart eines Katalysators in Gegenwart eines inerten Lösungsmittels bei Temperaturen zwischen −80 und +50°, vorzugsweise zwischen −20 und +30° und Drucken zwischen 1 und 2 bar. Als Katalysatoren eignen sich die meisten gebräuchlichen Hydrierkatalysatoren, vorzugsweise Palladiumkohle oder Raney-Nickel. Als Lösungsmittel können prinzipiell alle für solche Hydrierungen übliche Lösungsmittel verwendet werden. Besonders geeignet sind Kohlenwasserstoffe, vorzugsweise Benzol, Toluol oder Xylole und Alkohole, vorzugsweise Methanol, Ethanol, Isopropanol oder n-Propanol. Die Reaktion wird vorzugsweise in Gegenwart einer Base, vorzugsweise einem organischen Amin wie zum Beispiel Triethylamin, durchgeführt.

Die Verbindungen der Formel I sind ferner durch Diazotierung entsprechender Amine und anschließende Substitution der Diazoniumgruppen durch F-Atome, z. B. nach der Methode von Schiemann erhältlich.

Die Diazotierung, Umsetzung zum Diazoniumtetrafluoborat und thermische Zersetzung (Schiemann-Balz-Synthese) kann in an sich bekannter Weise durchgeführt werden, zum Beispiel nach einer der in «Organic Reactions», Band 5 (1949), Seiten 193–228 beschriebenen Verfahrensvarianten.

Die Ausgangsstoffe können z. B. durch Nitrierung entsprechender Pyrimidinderivate erhalten werden, die der Formel I entsprechen, aber an Stelle eines oder mehrerer F-Atome Wasserstoffatome enthalten; die erhaltenen Nitroverbindungen können anschließend, z. B. mit Zinn und Salzsäure oder durch katalytische Hydrierung, zu den Aminoverbindungen reduziert werden.

Die in den beschriebenen Synthesen eingesetzten Ausgangsverbindungen der Formeln II bis VII sind zum Teil bekannt, sie können alle in an sich üblicher Weise analog zu den bekannten Ausgangsverbindungen nach Standardverfahren der präparativen organischen Chemie hergestellt werden.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder -cyclohexyl-ester, Phenylcyclohexane, Cyclohexylbiphenyle, Cyclohexylcyclohexane, Cyclohexylnaphthaline, 1,4-Biscyclohexylbenzole, 4,4′-Bis-cyclohexylbiphenyle, Phenyl- oder Cyclohexylpyrimidine, Phenyl- oder Cyclohexyldioxane, ggf. halogenierten Stilbene, Benzylphenylether, Tolane und substituierten Zimtsäuren.

Die wichtigsten als Bestansteile derartiger flüssigkristalliner Dielektrika in Frage kommenden Verbindungen lassen sich durch die Formel VIII charakterisieren,

$$R'–E^1–G–E^2–R'' \qquad VIII$$

worin $E^1$ und $E^2$ je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | –CH=CH– | –N(O)=N– |
| | –CH=CY– | –CH=N(O)– |
| | –C≡C– | $-CH_2-CH_2-$ |
| | –CO–O– | $-CH_2-O-$ |
| | –CO–S– | $-CH_2-S-$ |
| | –CH=N– | –COO–Phe–COO– |

oder eine C–C-Einfachbindung, Y Halogen, vorzugsweise Chlor, oder –CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Die 1,4-disubstituierten Benzolringe in diesen Verbindungen können auch durch F oder CN lateral substituiert sein. Viele solcher Substanzen oder auch Gemische davon sind im Handel erhältlich, alle diese Substanzen sind nach literaturbekannten Methoden herstellbar.

Die erfindungsgemäßen Dielektrika enthalten etwa 0,1 bis 100, vorzugsweise 10 bis 100%, von Verbindungen der Formel I, wobei bei Abwesenheit anderer Verbindungen mindestens zwei Verbindungen der Formel I vorliegen.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z. B. I. Haller et al., Mol. Cryst. Liq. Cryst. Band 24, Seiten 249–258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS 2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben.

Beispiel 1

Man löst 53 g 2-p-Fluorphenyl-4,6-dichlor-5-n-hexylpyrimidin [erhältlich durch Umsetzung von p-Fluorbenzonitril mit Ethanol/HCl zu p-Fluorbenzimidoethylesterhydrochlorid, Umsetzung mit ethanolischem $NH_3$ zu p-Fluorbenzamidinhydrochlorid, Reaktion mit n-Hexylmalonsäurediethylester zu 2-p-Fluorphenyl-4,6-di-hydroxy-5-n-hexylpyrimidin und Erhitzen mit $POCl_3$/Dimethylanilin] in 750 ml Ethanol und 75 ml Triethylamin und hydriert an 25 g 5%igem Pd–C bei 20° und 1 bar, bis etwas mehr als die berechnete Menge Wasserstoff aufgenommen ist. Man filtriert, dampft ein, reinigt durch Lösen in Dichlormethan, Waschen mit Wasser, verdünnter Salzsäure und wieder mit Wasser, dampft erneut ein und erhält 2-p-Fluorphenyl-5-n-hexylpyrimidin, F. 39° (aus Ethanol).

Analog werden hergestellt:

2-p-Fluorphenyl-5-methylpyrimidin
2-p-Fluorphenyl-5-ethylpyrimidin
2-p-Fluorphenyl-5-n-propylpyrimidin
2-p-Fluorphenyl-5-n-butylpyrimidin
2-p-Fluorphenyl-5-n-pentylpyrimidin
2-p-Fluorphenyl-5-n-heptylpyrimidin
2-p-Fluorphenyl-5-n-octylpyrimidin
2-p-Fluorphenyl-5-n-nonylpyrimidin, F. 23,5°
2-p-Fluorphenyl-5-n-decylpyrimidin
2-p-Fluorphenyl-5-n-undecylpyrimidin
2-p-Fluorphenyl-5-n-dodecylpyrimidin

Beispiel 2

Man löst 8,8 g 2-(3-Amino-4-n-dodecyloxyphenyl)-5-n-hexylpyrimidin [F. 57,5°, erhältlich durch Umsetzung von 2-(4-n-Dodecyloxyphenyl)-5-n-hexylpyrimidin mit Salpetersäure zu 2-(3-Nitro-4-n-dodecyloxyphenyl)-5-n-hexylpyrimidin und nachfolgende Reduktion mit $H_2$/Raney-Nickel] bei 50° in 25 ml Dioxan und tropft unter Rühren 25 ml 35%ige Tetrafluorborsäure zu. Nach einer Stunde kühlt man die erhaltene Emulsion auf 0°, wobei Kristallisation auftritt. Die Suspension wird mit einer Lösung von 2,4 g Natriumnitrit in 25 ml Wasser versetzt und eine Stunde gerührt. Man saugt das erhaltene Diazoniumtetrafluoroborat ab, wäscht mit Eiswasser und trocknet. 5 g des Diazoniumsalzes werden bis zur Beendigung der Gasentwicklung auf 200° erhitzt. Durch chromatographische Aufreinigung erhält man 2-(3-Fluor-4-n-dodecyloxyphenyl)-5-n-hexylpyrimidin, F. 51,5°, K. 44°.

Analog werden hergestellt:

2-(3-Fluor-4-(trans-4-n-butylcyclohexyl)-phenyl)-5-n-butylpyrimidin
2-(3-Fluor-4-(trans-4-n-butylcyclohexyl)-phenyl)-5-n-pentylpyrimidin
2-(3-Fluor-4-(trans-4-n-butylcyclohexyl)-phenyl)-5-n-hexylpyrimidin
2-(3-Fluor-4-(trans-4-n-butylcyclohexyl)-phenyl)-5-n-heptylpyrimidin
2-(3-Fluor-4-(trans-4-n-butylcyclohexyl)-phenyl)-5-n-octylpyrimidin

2-(3-Fluor-4-(trans-4-n-pentylcyclohexyl)-phenyl)-5-n-butylpyrimidin
2-(3-Fluor-4-(trans-4-n-pentylcyclohexyl)-phenyl)-5-n-pentylpyrimidin
2-(3-Fluor-4-(trans-4-n-pentylcyclohexyl)-phenyl)-5-n-hexylpyrimidin
2-(3-Fluor-4-(trans-4-n-pentylcyclohexyl)-phenyl)-5-n-heptylpyrimidin
2-(3-Fluor-4-(trans-4-n-pentylcyclohexyl)-phenyl)-5-n-octylpyrimidin
2-(3-Fluor-4-(trans-4-n-hexylcyclohexyl)-phenyl)-5-n-butylpyrimidin
2-(3-Fluor-4-(trans-4-n-hexylcyclohexyl)-phenyl)-5-n-pentylpyrimidin
2-(3-Fluor-4-(trans-4-n-hexylcyclohexyl)-phenyl)-5-n-hexylpyrimidin
2-(3-Fluor-4-(trans-4-n-hexylcyclohexyl)-phenyl)-5-n-heptylpyrimidin
2-(3-Fluor-4-(trans-4-n-hexylcyclohexyl)-phenyl)-5-n-octylpyrimidin
2-(3-Fluor-4-(trans-4-n-heptylcyclohexyl)-phenyl)-5-n-butylpyrimidin
2-(3-Fluor-4-(trans-4-n-heptylcyclohexyl)-phenyl)-5-n-pentylpyrimidin
2-(3-Fluor-4-(trans-4-n-heptylcyclohexyl)-phenyl)-5-n-hexylpyrimidin
2-(3-Fluor-4-(trans-4-n-heptylcyclohexyl)-phenyl)-5-n-heptylpyrimidin
2-(3-Fluor-4-(trans-4-n-heptylcyclohexyl)-phenyl)-5-n-octylpyrimidin
2-(3-Fluor-4-(trans-4-n-octylcyclohexyl)-phenyl)-5-n-butylpyrimidin
2-(3-Fluor-4-(trans-4-n-octylcyclohexyl)-phenyl)-5-n-pentylpyrimidin
2-(3-Fluor-4-(trans-4-n-octylcyclohexyl)-phenyl)-5-n-hexylpyrimidin
2-(3-Fluor-4-(trans-4-n-octylcyclohexyl)-phenyl)-5-n-heptylpyrimidin
2-(3-Fluor-4-(trans-4-n-octylcyclohexyl)-phenyl)-5-n-octylpyrimidin
2-(3-Fluor-4-n-butylphenyl)-5-(4-n-butylphenyl)-pyrimidin
2-(3-Fluor-4-n-butylphenyl)-5-(4-n-pentylphenyl)-pyrimidin
2-(3-Fluor-4-n-butylphenyl)-5-(4-n-hexylphenyl)-pyrimidin
2-(3-Fluor-4-n-butylphenyl)-5-(4-n-heptylphenyl)-pyrimidin
2-(3-Fluor-4-n-butylphenyl)-5-(4-n-octylphenyl)-pyrimidin
2-(3-Fluor-4-n-pentylphenyl)-5-(4-n-butylphenyl)-pyrimidin
2-(3-Fluor-4-n-pentylphenyl)-5-(4-n-pentylphenyl)-pyrimidin
2-(3-Fluor-4-n-pentylphenyl)-5-(4-n-hexylphenyl)-pyrimidin
2-(3-Fluor-4-n-pentylphenyl)-5-(4-n-heptylphenyl)-pyrimidin
2-(3-Fluor-4-n-pentylphenyl)-5-(4-n-octylphenyl)-pyrimidin
2-(3-Fluor-4-n-hexylphenyl)-5-(4-n-butylphenyl)-pyrimidin
2-(3-Fluor-4-n-hexylphenyl)-5-(4-n-pentylphenyl)-pyrimidin
2-(3-Fluor-4-n-hexylphenyl)-5-(4-n-hexylphenyl)-pyrimidin
2-(3-Fluor-4-n-hexylphenyl)-5-(4-n-heptylphenyl)-pyrimidin
2-(3-Fluor-4-n-hexylphenyl)-5-(4-n-octylphenyl)-pyrimidin
2-(3-Fluor-4-n-heptylphenyl)-5-(4-n-butylphenyl)-pyrimidin
2-(3-Fluor-4-n-heptylphenyl)-5-(4-n-pentylphenyl)-pyrimidin
2-(3-Fluor-4-n-heptylphenyl)-5-(4-n-hexylphenyl)-pyrimidin
2-(3-Fluor-4-n-heptylphenyl)-5-(4-n-heptylphenyl)-pyrimidin
2-(3-Fluor-4-n-heptylphenyl)-5-(4-n-octylphenyl)-pyrimidin
2-(3-Fluor-4-n-octylphenyl)-5-(4-n-butylphenyl)-pyrimidin
2-(3-Fluor-4-n-octylphenyl)-5-(4-n-pentylphenyl)-pyrimidin
2-(3-Fluor-4-n-octylphenyl)-5-(4-n-hexylphenyl)-pyrimidin
2-(3-Fluor-4-n-octylphenyl)-5-(4-n-heptylphenyl)-pyrimidin
2-(3-Fluor-4-n-octylphenyl)-5-(4-n-octylphenyl)-pyrimidin
2-(3-Fluor-4-n-butoxyphenyl)-5-n-butylpyrimidin
2-(3-Fluor-4-n-butoxyphenyl)-5-n-pentylpyrimidin
2-(3-Fluor-4-n-butoxyphenyl)-5-n-hexylpyrimidin
2-(3-Fluor-4-n-butoxyphenyl)-5-n-heptylpyrimidin
2-(3-Fluor-4-n-butoxyphenyl)-5-n-octylpyrimidin
2-(3-Fluor-4-n-pentyloxyphenyl)-5-n-butylpyrimidin
2-(3-Fluor-4-n-pentyloxyphenyl)-5-n-pentylpyrimidin
2-(3-Fluor-4-n-pentyloxyphenyl)-5-n-hexylpyrimidin
2-(3-Fluor-4-n-pentyloxyphenyl)-5-n-heptylpyrimidin
2-(3-Fluor-4-n-pentyloxyphenyl)-5-n-octylpyrimidin
2-(3-Fluor-4-n-hexyloxyphenyl)-5-n-butylpyrimidin
2-(3-Fluor-4-n-hexyloxyphenyl)-5-n-pentylpyrimidin
2-(3-Fluor-4-n-hexyloxyphenyl)-5-n-hexylpyrimidin
2-(3-Fluor-4-n-hexyloxyphenyl)-5-n-heptylpyrimidin
2-(3-Fluor-4-n-hexyloxyphenyl)-5-n-octylpyrimidin
2-(3-Fluor-4-n-heptyloxyphenyl)-5-n-butylpyrimidin
2-(3-Fluor-4-n-heptyloxyphenyl)-5-n-pentylpyrimidin
2-(3-Fluor-4-n-heptyloxyphenyl)-5-n-hexylpyrimidin
2-(3-Fluor-4-n-heptyloxyphenyl)-5-n-heptylpyrimidin
2-(3-Fluor-4-n-heptyloxyphenyl)-5-n-octylpyrimidin
2-(3-Fluor-4-n-octyloxyphenyl)-5-n-butylpyrimidin
2-(3-Fluor-4-n-octyloxyphenyl)-5-n-pentylpyrimidin
2-(3-Fluor-4-n-octyloxyphenyl)-5-n-hexylpyrimidin

2-(3-Fluor-4-n-octyloxyphenyl)-5-n-heptylpyrimidin
2-(3-Fluor-4-n-octyloxyphenyl)-5-n-octylpyrimidin

Beispiel 3

Man löst 56 g 2-(3-Amino-4-n-pentylphenyl)-5-n-hexylpyrimidin [F. 92°, erhältlich durch Umsetzung von 2-(4-n-Pentylphenyl)-5-n-hexylpyrimidin (K. 28,5°) mit Salpetersäure zu 2-(3-Nitro-4-n-pentylphenyl)-5-n-hexylpyrimidin (F. 41,5°) und Hydrieren an Raney-Nickel] bei 50° in 150 ml Dioxan und tropft unter Rühren 208 ml 35%ige Tetrafluorborsäure zu. Nach einer Stunde kühlt man die erhaltenen Emulsion auf 0°, wobei Kristallisation auftritt. Die Suspension wird mit einer Lösung von 20 g Natriumnitrit in 150 ml Wasser versetzt und eine Stunde gerührt. Man saugt das erhaltene Diazoniumtetrafluoroborat ab, wäscht mit Eiswasser und trocknet. 5 g des Diazoniumsalzes werden bis zur Beendigung der Gasentwicklung auf 200° erhitzt. Durch chromatographische Aufreinigung erhält man 2-(3-Fluor-4-n-pentylphenyl)-5-n-hexylpyrimidin, F. 30°.

Analog werden hergestellt:

2-(3-Fluor-4-n-butylphenyl)-5-(trans-4-n-butylcyclohexyl)-pyrimidin
2-(3-Fluor-4-n-butylphenyl)-5-(trans-4-n-pentylcyclohexyl)-pyrimidin
2-(3-Fluor-4-n-butylphenyl)-5-(trans-4-n-hexylcyclohexyl)-pyrimidin
2-(3-Fluor-4-n-butylphenyl)-5-(trans-4-n-heptylcyclohexyl)-pyrimidin
2-(3-Fluor-4-n-butylphenyl)-5-(trans-4-n-octylcyclohexyl)-pyrimidin
2-(3-Fluor-4-n-pentylphenyl)-5-(trans-4-n-butylcyclohexyl)-pyrimidin
2-(3-Fluor-4-n-pentylphenyl)-5-(trans-4-n-pentylcyclohexyl)-pyrimidin
2-(3-Fluor-4-n-pentylphenyl)-5-(trans-4-n-hexylcyclohexyl)-pyrimidin
2-(3-Fluor-4-n-pentylphenyl)-5-(trans-4-n-heptylcyclohexyl)-pyrimidin
2-(3-Fluor-4-n-pentylphenyl)-5-(trans-4-n-octylcyclohexyl)-pyrimidin
2-(3-Fluor-4-n-hexylphenyl)-5-(trans-4-n-butylcyclohexyl)-pyrimidin
2-(3-Fluor-4-n-hexylphenyl)-5-(trans-4-n-pentylcyclohexyl)-pyrimidin
2-(3-Fluor-4-n-hexylphenyl)-5-(trans-4-n-hexylcyclohexyl)-pyrimidin
2-(3-Fluor-4-n-hexylphenyl)-5-(trans-4-n-heptylcyclohexyl)-pyrimidin
2-(3-Fluor-4-n-hexylphenyl)-5-(trans-4-n-octylcyclohexyl)-pyrimidin
2-(3-Fluor-4-n-heptylphenyl)-5-(trans-4-n-butylcyclohexyl)-pyrimidin
2-(3-Fluor-4-n-heptylphenyl)-5-(trans-4-n-pentylcyclohexyl)-pyrimidin
2-(3-Fluor-4-n-heptylphenyl)-5-(trans-4-n-hexylcyclohexyl)-pyrimidin
2-(3-Fluor-4-n-heptylphenyl)-5-(trans-4-n-heptylcyclohexyl)-pyrimidin
2-(3-Fluor-4-n-heptylphenyl)-5-(trans-4-n-octylcyclohexyl)-pyrimidin
2-(3-Fluor-4-n-octylphenyl)-5-(trans-4-n-butylcyclohexyl)-pyrimidin
2-(3-Fluor-4-n-octylphenyl)-5-(trans-4-n-pentylcyclohexyl)-pyrimidin
2-(3-Fluor-4-n-octylphenyl)-5-(trans-4-n-hexylcyclohexyl)-pyrimidin
2-(3-Fluor-4-n-octylphenyl)-5-(trans-4-n-heptylcyclohexyl)-pyrimidin
2-(3-Fluor-4-n-octylphenyl)-5-(trans-4-n-octylcyclohexyl)-pyrimidin
2-(3-Fluor-4-n-butylphenyl)-5-n-butylpyrimidin
2-(3-Fluor-4-n-butylphenyl)-5-n-pentylpyrimidin
2-(3-Fluor-4-n-butylphenyl)-5-n-hexylpyrimidin
2-(3-Fluor-4-n-butylphenyl)-5-n-heptylpyrimidin
2-(3-Fluor-4-n-butylphenyl)-5-n-octylpyrimidin
2-(3-Fluor-4-n-pentylphenyl)-5-n-butylpyrimidin
2-(3-Fluor-4-n-pentylphenyl)-5-n-pentylpyrimidin
2-(3-Fluor-4-n-pentylphenyl)-5-n-hexylpyrimidin
2-(3-Fluor-4-n-pentylphenyl)-5-n-heptylpyrimidin
2-(3-Fluor-4-n-pentylphenyl)-5-n-octylpyrimidin
2-(3-Fluor-4-n-hexylphenyl)-5-n-butylpyrimidin
2-(3-Fluor-4-n-hexylphenyl)-5-n-pentylpyrimidin
2-(3-Fluor-4-n-hexylphenyl)-5-n-hexylpyrimidin
2-(3-Fluor-4-n-hexylphenyl)-5-n-heptylpyrimidin
2-(3-Fluor-4-n-hexylphenyl)-5-n-octylpyrimidin
2-(3-Fluor-4-n-heptylphenyl)-5-n-butylpyrimidin
2-(3-Fluor-4-n-heptylphenyl)-5-n-pentylpyrimidin
2-(3-Fluor-4-n-heptylphenyl)-5-n-hexylpyrimidin
2-(3-Fluor-4-n-heptylphenyl)-5-n-heptylpyrimidin
2-(3-Fluor-4-n-heptylphenyl)-5-n-octylpyrimidin
2-(3-Fluor-4-n-octylphenyl)-5-n-butylpyrimidin
2-(3-Fluor-4-n-octylphenyl)-5-n-pentylpyrimidin
2-(3-Fluor-4-n-octylphenyl)-5-n-hexylpyrimidin
2-(3-Fluor-4-n-octylphenyl)-5-n-heptylpyrimidin
2-(3-Fluor-4-n-octylphenyl)-5-n-octylpyrimidin
2-Cyano-5-(3-Fluor-4-n-butylphenyl)-pyrimidin
2-Cyano-5-(3-Fluor-4-n-pentylphenyl)-pyrimidin
2-Cyano-5-(3-Fluor-4-n-hexylphenyl)-pyrimidin
2-Cyano-5-(3-Fluor-4-n-heptylphenyl)-pyrimidin
2-Cyano-5-(3-Fluor-4-n-octylphenyl)-pyrimidin

Beispiel 4

Man löst 82,6 g 2-(2′-Fluor-4′-n-pentyl-biphenyl-4-yl)-4,6-dichlor-5-n-nonylpyrimidin [F. 51°, erhältlich durch Umsetzung von 4-n-Pentyl-2-fluor-4′-cyanobiphenyl, F. 54°, mit Ethanol/HCl und ethanolischem $NH_3$ zum entsprechenden Amidinhydrochlorid, Reaktion mit n-Nonylmalonsäurediethylester zu 2-(2′-Fluor-4′-n-pentylbiphenyl-4-yl)-4,6-dihydroxy-5-n-nonylpyrimidin und Erhitzen mit $POCl_3$/Dimethylanilin] in 750 ml Ethanol und 75 ml Triethylamin und hydriert und arbeitet auf analog zu Beispiel 1. Man erhält 2-(2′-Fluor-4′-n-pentylbiphenyl-4-yl)-5-n-nonylpyrimidin, F. 51°, S/N 81°, K. 131°.

Analog werden hergestellt:

2-(2′-Fluor-4′-n-butylbiphenyl-4-yl)-5-n-butylpyrimidin
2-(2′-Fluor-4′-n-butylbiphenyl-4-yl)-5-n-pentylpyrimidin
2-(2′-Fluor-4′-n-butylbiphenyl-4-yl)-5-n-hexylpyrimidin
2-(2′-Fluor-4′-n-butylbiphenyl-4-yl)-5-n-heptylpyrimidin

2-(2'-Fluor-4'-n-butylbiphenyl-4-yl)-5-n-octylpyrimidin
2-(2'-Fluor-4'-n-butylbiphenyl-4-yl)-5-n-nonylpyrimidin
2-(2'-Fluor-4'-n-pentylbiphenyl-4-yl)-5-n-butylpyrimidin
2-(2'-Fluor-4'-n-pentylbiphenyl-4-yl)-5-n-pentylpyrimidin
2-(2'-Fluor-4'-n-pentylbiphenyl-4-yl)-5-n-hexylpyrimidin
2-(2'-Fluor-4'-n-pentylbiphenyl-4-yl)-5-n-heptylpyrimidin
2-(2'-Fluor-4'-n-pentylbiphenyl-4-yl)-5-n-octylpyrimidin
2-(2'-Fluor-4'-n-pentylbiphenyl-4-yl)-5-n-decylpyrimidin
2-(2'-Fluor-4'-n-pentylbiphenyl-4-yl)-5-n-undecylpyrimidin
2-(2'-Fluor-4'-n-pentylbiphenyl-4-yl)-5-n-dodecylpyrimidin
2-(2'-Fluor-4'-n-hexylbiphenyl-4-yl)-5-n-butylpyrimidin
2-(2'-Fluor-4'-n-hexylbiphenyl-4-yl)-5-n-pentylpyrimidin
2-(2'-Fluor-4'-n-hexylbiphenyl-4-yl)-5-n-hexylpyrimidin
2-(2'-Fluor-4'-n-hexylbiphenyl-4-yl)-5-n-heptylpyrimidin
2-(2'-Fluor-4'-n-hexylbiphenyl-4-yl)-5-n-octylpyrimidin
2-(2'-Fluor-4'-n-hexylbiphenyl-4-yl)-5-n-nonylpyrimidin
2-(2'-Fluor-4'-n-heptylbiphenyl-4-yl)-5-n-butylpyrimidin
2-(2'-Fluor-4'-n-heptylbiphenyl-4-yl)-5-n-pentylpyrimidin
2-(2'-Fluor-4'-n-heptylbiphenyl-4-yl)-5-n-hexylpyrimidin
2-(2'-Fluor-4'-n-heptylbiphenyl-4-yl)-5-n-heptylpyrimidin
2-(2'-Fluor-4'-n-heptylbiphenyl-4-yl)-5-n-octylpyrimidin
2-(2'-Fluor-4'-n-octylbiphenyl-4-yl)-5-n-butylpyrimidin
2-(2'-Fluor-4'-n-octylbiphenyl-4-yl)-5-n-pentylpyrimidin
2-(2'-Fluor-4'-n-octylbiphenyl-4-yl)-5-n-hexylpyrimidin
2-(2'-Fluor-4'-n-octylbiphenyl-4-yl)-5-n-heptylpyrimidin
2-(2'-Fluor-4'-n-octylbiphenyl-4-yl)-5-n-octylpyrimidin
2-(2-Fluor-biphenyl-4-yl)-5-n-butylpyrimidin
2-(2-Fluor-biphenyl-4-yl)-5-n-pentylpyrimidin
2-(2-Fluor-biphenyl-4-yl)-5-n-hexylpyrimidin
2-(2-Fluor-biphenyl-4-yl)-5-n-heptylpyrimidin
2-(2-Fluor-biphenyl-4-yl)-5-n-octylpyrimidin
2-(2-Fluor-4'-n-pentylbiphenyl-4-yl)-5-n-butylpyrimidin
2-(2-Fluor-4'-n-pentylbiphenyl-4-yl)-5-n-pentylpyrimidin
2-(2-Fluor-4'-n-pentylbiphenyl-4-yl)-5-n-hexylpyrimidin
2-(2-Fluor-4'-n-pentylbiphenyl-4-yl)-5-n-heptylpyrimidin
2-(2-Fluor-4'-n-pentylbiphenyl-4-yl)-5-n-octylpyrimidin
2-(2-Fluor-4'-n-hexylbiphenyl-4-yl)-5-n-butylpyrimidin
2-(2-Fluor-4'-n-hexylbiphenyl-4-yl)-5-n-pentylpyrimidin
2-(2-Fluor-4'-n-hexylbiphenyl-4-yl)-5-n-hexylpyrimidin
2-(2-Fluor-4'-n-hexylbiphenyl-4-yl)-5-n-heptylpyrimidin
2-(2-Fluor-4'-n-hexylbiphenyl-4-yl)-5-n-octylpyrimidin
2-(2-Fluor-4'-n-heptylbiphenyl-4-yl)-5-n-butylpyrimidin
2-(2-Fluor-4'-n-heptylbiphenyl-4-yl)-5-n-pentylpyrimidin
2-(2-Fluor-4'-n-heptylbiphenyl-4-yl)-5-n-hexylpyrimidin
2-(2-Fluor-4'-n-heptylbiphenyl-4-yl)-5-n-heptylpyrimidin
2-(2-Fluor-4'-n-heptylbiphenyl-4-yl)-5-n-octylpyrimidin

Beispiel A

Eine flüssigkristalline Mischung aus

| | |
|---|---|
| 14,2% | 4-(trans-4-n-Propylcyclohexyl)-benzonitril |
| 19,2% | 4-(trans-4-n-Pentylcyclohexyl)-benzonitril |
| 13,3% | 4-(trans-4-n-Propylcyclohexyl)-ethoxybenzol |
| 10,0% | 4-(trans-4-n-Propylcyclohexyl)-butoxybenzol |
| 18,4% | 4-(trans-4-n-Pentylcyclohexyl)-4'-ethylbiphenyl |
| 8,3% | 4-(trans-4-n-Pentylcyclohexyl)-4'-(trans-4-n-propylcyclohexyl)-biphenyl und |
| 16,6% | 2-(4-n-Pentylphenyl)-5-n-hexylpyrimidin |

hat einen Phasenübergang smektisch/nematisch bei −12° und einen Klärpunkt von 72°. Durch Ersatz der letzten Komponente durch die gleiche Menge an 2-(3-Fluor-4-n-pentylphenyl)-5-n-hexylpyrimidin erhält man eine flüssigkristalline Mischung mit einem Klärpunkt von 65°, die bis −20° keine smektische Phase aufweist.

Beispiel B

Eine flüssigkristalline Mischung aus

| | |
|---|---|
| 12,6% | 4-(trans-4-n-Propylcyclohexyl)-benzonitril |
| 17,0% | 4-(trans-4-n-Pentylcyclohexyl)-benzonitril |
| 11,8% | 4-(trans-4-n-Propylcyclohexyl)-ethoxybenzol |
| 8,9% | 4-(trans-4-n-Propylcyclohexyl)-butoxybenzol |
| 16,3% | 4-(trans-4-n-Pentylcyclohexyl)-4'-ethylbiphenyl |
| 7,4% | 4-(trans-4-n-Pentylcyclohexyl)-4'-(trans-4-n-propylcyclohexyl)-biphenyl und |
| 26,0% | 2-(4-n-Pentylphenyl)-5-n-hexylpyrimidin |

hat einen Phasenübergang smektisch/nematisch bei +24° und einen Klärpunkt von 63°.

Durch Ersatz der letzten Komponente durch die gleiche Menge an 2-(3-Fluor-4-n-pentylphenyl)-5-n-hexylpyrimidin erhält man eine flüssigkristalline Mischung mit einem nematischen Phasenbereich von +11° bis +53°.

Beispiel C
Eine flüssigkristalline Mischung aus

16,7% 4-(trans-4-Ethylcyclohexyl)-benzonitril
8,3% 4-(trans-4-n-Butylcyclohexyl)-benzonitril
10,8% 4-Ethyl-4′-cyanobiphenyl
18,3% 4-n-Butyl-4′-cyanobiphenyl
16,7% trans-4-n-Butylcyclohexancarbonsäure-4-(trans-4-n-propylcyclohexyl)-phenylester
12,5% trans-4-n-Penylcyclohexancarbonsäure-4-(trans-4-n-propylcyclohexyl)-phenylester und
16,7% 2-(4-n-Pentylphenyl)-5-n-hexylpyrimidin

hat einen Phasenübergang smektisch/nematisch bei −8° und einen Klärpunkt von 58°. Durch Ersatz der letzten Komponente durch die gleiche Menge an 2-(3-Fluor-4-n-pentylphenyl)1-5-n-hexylpyrimidin erhält man eine flüssigkristalline Mischung mit einem nematischen Phasenbereich von −18° bis +52°.

Beispiel D
Eine flüssigkristalline Mischung aus

14,8% 4-(trans-4-Ethylcyclohexyl)-benzonitril
7,4% 4-(trans-4-n-Butylcyclohexyl)-benzonitril
9,6% 4-Ethyl-4′-cyanobiphenyl
16,4% 4-n-Butyl-4′-cyanobiphenyl
14,8% trans-4-n-Butylcyclohexancarbonsäure-4-(trans-4-n-propylcyclohexyl)-phenylester
11,1% trans-4-n-Pentylcyclohexancarbonsäure-4-(trans-4-n-propylcyclohexyl)-phenylester und
25,9% 2-(4-n-Pentylphenyl)-5-n-hexylpyrimidin

hat einen Phasenübergang smektisch/nematisch bei +15° und einen Klärpunkt von 51°.

Durch Ersatz der letzten Komponente durch die gleiche Menge an 2-(3-Fluor-4-n-pentylphenyl)-5-n-hexylpyrimidin erhält man eine flüssigkristalline Mischung mit einem nematischen Phasenbereich von +1° bis +41°.

Beispiel E
Eine flüssigkristalline Mischung aus

12,6% 4-(trans-5-n-Propyl-1,3-dioxan-2-yl)-benzonitril
17,0% 4-(trans-5-n-Butyl-1,3-dioxan-2-yl)-benzonitril
10,4% 4-Ethyl-4′-cyanobiphenyl
19,2% 4-n-Butyl-4′-cyanobiphenyl
5,9% 4-(trans-4-n-Pentylcyclohexyl)-4′-cyanobiphenyl
3,7% 4-(trans-4-Ethylcyclohexyl)-benzoesäure-(4-cyanophenyl)-ester
5,2% 4-(trans-4-n-Pentylcyclohexyl)-benzoesäure-(4-cyanophenyl)-ester und
26,0% 2-(4-n-Pentylphenyl)-5-n-hexylpyrimidin

hat einen Phasenübergang smektisch/nematisch bei 0° und einen Klärpunkt von 47°.

Durch Ersatz der letzten Komponente durch die gleiche Menge an 2-(3-Fluor-4-n-pentylphenyl)-5-n-hexylpyrimidin erhält man eine flüssigkristalline Mischung mit einem nematischen Phasenbereich von −20° bis +36°.

Beispiel F
Eine flüssigkristalline Mischung aus

11,1% 4-(trans-4-n-Propylcyclohexyl)-benzonitril
20,0% 4-(trans-4-n-Propylcyclohexyl)-ethylbenzol
7,4% 4-(trans-4-n-Propylcyclohexyl)-ethoxybenzol
5,2% 4-(trans-4-n-Pentylcyclohexyl)-4′-cyanobiphenyl
6,7% 4-(trans-4-n-Propylcyclohexyl)-4′-ethylbiphenyl
5,9% 4-(trans-4-n-Pentylcyclohexyl)-4′-ethylbiphenyl
7,4% 4-(trans-4-n-Pentylcyclohexyl)-4′-(trans-4-n-propylcyclohexyl)-biphenyl
5,9% 4-(trans-4-n-Propylcyclohexyl)-benzoesäure-(4-n-propylphenyl)-ester
4,4% 4-(trans-4-n-Pentylcyclohexyl)-benzoesäure-(4-n-propylphenyl)-ester und
26,0% 2-(4-n-Pentylphenyl)-5-n-hexylpyrimidin

hat einen Phasenübergang smektisch/nematisch bei −17° und einen Klärpunkt von 58°.

Durch Ersatz der letzten Komponente durch die gleiche Menge an 2-(3-Fluor-4-n-pentylphenyl)-5-n-hexylpyrimidin erhält man eine flüssigkristalline Mischung mit einem nematischen Phasenbereich von < −20° bis +48°.

Beispiel G
Eine flüssigkristalline Mischung aus

9% 4-(trans-5-n-Propyl-1,3-dioxan-2-yl)-benzonitril
12% 4-(trans-5-n-Butyl-1,3-dioxan-2-yl)-benzonitril
9% 4-(trans-5-n-Pentyl-1,3-dioxan-2-yl)-benzonitril
6% 2-(4-n-Heptyloxyphenyl)-5-n-hexylpyrimidin
22% 2-(4-n-Octyloxyphenyl)-5-n-hexylpyrimidin
6% 2-(4-n-Nonyloxyphenyl)-5-n-hexylpyrimidin
6% 2-(4-n-Decyloxyphenyl)-5-n-hexylpyrimidin
10% trans-4-n-Propylcyclohexancarbonsäure-4-ethoxyphenylester
10% trans-4-n-Butylcyclohexancarbonsäure-4-ethoxyphenylester und
10% 4-(trans-4-n-Propylcyclohexyl)-4′-ethylbiphenyl

hat einen Schmelzpunkt von −10°, einen Klärpunkt von 61° und einen Phasenübergang smektisch/nematisch bei +21°.

Durch Ersatz von 13% des in der obigen Mischung enthaltenen Pyrimidinanteils durch 2-(3-Fluor-4-n-dodecyloxyphenyl)-5-n-hexylpyrimidin

erhält man eine flüssigkristalline Mischung mit einem Schmelzpunkt von −12°, einem Klärpunkt von 58° und einem Phasenübergang smektisch/nematisch unterhalb −20°. Die Mischung zeichnet sich durch eine günstige Schwellenspannung und eine steile Kennlinie (kleines Verhältnis $V_{50}/V_{10}$) aus und eignet sich somit vorzüglich zum Multiplexbetrieb.

**Patentansprüche**

1. Fluorhaltige Pyrimidinverbindungen der Formel I

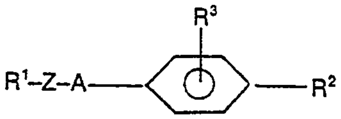
I

worin
$R^1$ und $R^2$ H, R oder OR bedeuten, wobei einer davon auch CN oder F, oder, sofern A eine kovalente Bindung ist, auch 4-$R^4$-Phenyl oder 4-R-Cyclohexyl bedeuten, wobei $R^4$ R, OR, CN oder F bedeutet und R eine Alkylgruppe mit 1 bis 12 C-Atomen ist,
$R^3$ F oder, wenn einer der Reste $R^1$ oder $R^2$ F oder 4-Fluorphenyl ist, auch H bedeutet,
Z 1,3-Pyrimidin-2,5-diyl und
A 1,4-Phenylen, 1,4-Cyclohexylen oder eine kovalente Bindung ist,
sowie der Formeln

| | |
|---|---|
| R–Ph–Z–PhF–CN | I dj |
| RO–Ph–Z–PhF–CN | I dk |
| R–Ph–Z–Ph–F | I dm |
| RO–Ph–Z–Ph–F | I dn |
| F–Z–Ph–Cy–R | I l |
| R–Cy–Z–Ph–F | I n |
| R–Cy–Z–PhF–F | I od |
| R–Cy–Z–PhF–CN | I oe |
| F–Z–PhF–Cy–R | I pc |
| NC–Z–PhF–Cy–R | I pd |
| R–Z–PhF–Ph–CN | I qd |
| R–Z–PhF–Ph–F | I qe |
| NC–Z–PhF–Ph–R | I qf |
| NC–Z–PhF–Ph–OR | I qg |

worin
Ph 1,4-Phenylen,
PhF 2- bzw. 3-Fluor-1,4-phenylen,
Cy 1,4-Cyclohexylen,
R eine Alkylgruppe mit 1 bis 12 C-Atomen, und
Z 1,3-Pyrimidin-2,5-diyl ist.

2. Pyrimidinverbindungen nach Anspruch 1, gekennzeichnet durch die Formeln I a und I c

| | |
|---|---|
| $R^1$–Z–Ph–F | I a |
| $R^1$–Z–Ph–Ph–F | I c |

worin
$R^1$ geradkettiges Alkyl mit 2 bis 8 Kohlenstoffatomen,
Z 1,3-Pyrimidin-2,5-diyl und
Ph 1,4-Phenylen bedeutet.

3. Pyrimidinverbindungen nach Anspruch 1, gekennzeichnet durch die Formeln I bc, I dj, I fd, I jd und I oe,

| | |
|---|---|
| R–Z–PhF–CN | I bc |
| R–Ph–Z–PhF–CN | I dj |
| R–Z–Ph–PhF–CN | I fd |
| R–Z–Cy–PhF–CN | I jd |
| R–Cy–Z–PhF–CN | I oe |

worin
R geradkettiges Alkyl mit 2 bis 8 Kohlenstoffatomen,
Z 1,3-Pyrimidin-2,5-diyl,
Ph 1,4-Phenylen,
Cy 1,4-Cyclohexylen und
PhF 2- bzw. 3-Fluor-1,4-phenylen bedeutet.

4. Pyrimidinverbindungen nach Anspruch 1, gekennzeichnet durch die Formeln I bd, I fe, I je und I od,

| | |
|---|---|
| R–Z–PhF–F | I bd |
| R–Z–Ph–PhF–F | I fe |
| R–Z–Cy–PhF–F | I je |
| R–Cy–Z–PhF–F | I od |

worin
R geradkettiges Alkyl mit 2 bis 8 Kohlenstoffatomen,
Z 1,3-Pyrimidin-2,5-diyl,
Ph 1,4-Phenylen,
Cy 1,4-Cyclohexylen und
PhF 2- bzw. 3-Fluor-1,4-phenylen bedeutet.

5. Pyrimidinverbindungen nach Anspruch 1, dadurch gekennzeichnet, daß einer der Reste $R^1$ und $R^2$ trans-4-R-Cyclohexyl ist.

6. Pyrimidinverbindungen nach Anspruch 1, dadurch gekennzeichnet, daß einer der Reste $R^1$ und $R^2$ Alkyl und der andere CN bedeutet.

7. Verfahren zur Herstellung von fluorhaltigen Pyrimidinverbindungen der Formel I nach Anspruch 1, dadurch gekennzeichnet, daß man
(a) ein Halogenpyrimidin der Formel II

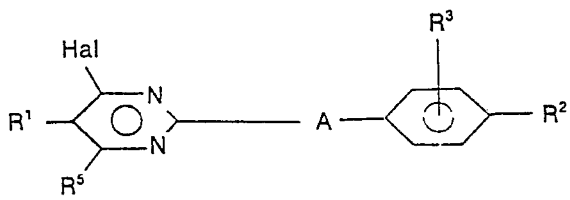
II

worin
$R^5$ H oder Hal und
Hal Cl oder Br bedeuten und
$R^1$, $R^2$, $R^3$ und A die angegebene Bedeutung haben, reduktiv dehalogeniert oder daß man
(b) eine Verbindung, die der Formel I entspricht, jedoch an Stelle eines oder mehrerer Fluoratome eine oder mehrere Aminogruppen enthält, diazotiert, in das entsprechende Diazoniumtetrafluoroborat überführt und dieses thermisch zersetzt.

8. Verwendung der Verbindungen nach einem der Ansprüche 1 bis 6 als Komponenten flüssigkristalliner Dielektrika für elektrooptische Anzeigeelemente.

9. Flüssigkristallines Dielektrikum für elektrooptische Anzeigeelemente mit mindestens zwei flüssigkristallinen Komponenten, dadurch gekennzeichnet, daß mindestens eine Komponente eine Verbindung nach einem der Ansprüche 1 bis 6 ist.

10. Elektrooptisches Anzeigeelement, dadurch gekennzeichnet, daß es ein Dielektrikum nach Anspruch 9 enthält.

## Claims

1. Fluorine-containing pyrimidine compounds of the formula I

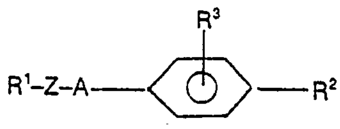

I

wherein $R^1$ and $R^2$ are H, R or OR, wherein one of them is also CN or F, or, if A is a covalent bond, also 4-$R^4$-phenyl or 4-R-cyclohexyl, wherein $R^4$ is R, OR, CN or F and R is an alkyl group with 1 to 12 C atoms, $R^3$ is F or, if one of the radicals $R^1$ or $R^2$ is F or 4-fluorophenyl, also H, Z is 1,3-pyrimidin-2,5-diyl and A is 1,4-phenylene, 1,4-cyclohexylene or a covalent bond and also of the formulae

| | |
|---|---|
| R–Ph–Z–PhF–CN | I dj |
| RO–Ph–Z–PhF–CN | I dk |
| R–Ph–Z–Ph–F | I dm |
| RO–Ph–Z–Ph–F | I dn |
| F–Z–Ph–Cy–R | I l |
| R–Cy–Z–Ph–F | I n |
| R–Cy–Z–PhF–F | I od |
| R–Cy–Z–PhF–CN | I oe |
| F–Z–PhF–Cy–R | I pc |
| NC–Z–PhF–Cy–R | I pd |
| R–Z–PhF–Ph–CN | I qd |
| R–Z–PhF–Ph–F | I qe |
| NC–Z–PhF–Ph–R | I qf |
| NC–Z–PhF–Ph–OR | I qg |

wherein
Ph is 1,4-phenylene,
PhF is 2- or 3-fluoro-1,4-phenylene,
Cy is 1,4-cyclohexylene,
R is an alkyl group with 1 to 12 C atoms, and
Z is 1,3-pyrimidine-2,5-diyl.

2. Pyrimidine compounds according to Claim 1, characterized by the formulae I a and I c

| | |
|---|---|
| $R^1$–Z–Ph–F | I a |
| $R^1$–Z–Ph–Ph–F | I c |

wherein
$R^1$ is straight-chain alkyl with 2 to 8 carbon atoms,
Z is 1,3-pyrimidine-2,5-diyl and
Ph is 1,4-phenylene.

3. Pyrimidine compounds according to Claim 1, characterized by the formulae I bc, I dj, I fd, I jd and I oe,

| | |
|---|---|
| R–Z–PhF–CN | I bc |
| R–Ph–Z–PhF–CN | I dj |
| R–Z–Ph–PhF–CN | I fd |
| R–Z–Cy–PhF–CN | I jd |
| R–Cy–Z–PhF–CN | I oe |

wherein
R is straight-chain alkyl with 2 to 8 carbon atoms,
Z is 1,3-pyrimidine-2,5-diyl,
Ph is 1,4-Phenylene,
Cy is 1,4-cyclohexylene and
PhF is 2- or 3-fluoro-1,4-phenylene.

4. Pyrimidine compounds according to Claim 1, characterized by the formulae I bd, I fe, I je and I od,

| | |
|---|---|
| R–Z–PhF–F | I bd |
| R–Z–Ph–PhF–F | I fe |
| R–Z–Cy–PhF–F | I je |
| R–Cy–PhF–F | I od |

wherein
R is straight-chain alkyl with 2 to 8 carbon atoms,
Z is 1,3-pyrimidine-2,5-diyl,
Ph is 1,4-phenylene,
Cy is 1,4-cyclohexylene and
PhF is 2- or 3-fluoro-1,4-phenylene.

5. Pyrimidine compounds according to Claim 1, characterized in that one of the radicals $R^1$ and $R^2$ is trans-4-R-cyclohexyl.

6. Pyrimidine compounds according to Claim 1, characterized in that one of the radicals $R^1$ and $R^2$ is alkyl and the other is CN.

7. Process for the preparation of fluorine-containing pyrimidine compounds of the formula I according to Claim 1, characterized in that

(a) a halogenopyrimidine of the formula II

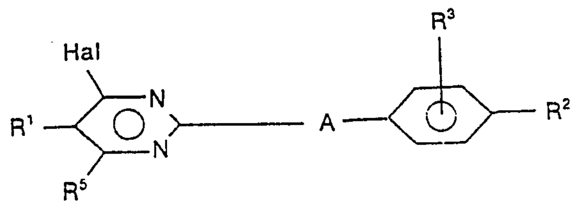

II

wherein $R^5$ is H or Hal and Hal is Cl or Br, and $R^1$, $R^2$, $R^3$ and A have the meaning given, is dehalogenated by reduction, or in that

(b) a compound which corresponds to the formula I but contains one or more amino groups instead of one or more fluorine atoms is diazotised, the diazotisation product is converted into the corresponding diazonium tetrafluoborate and this is decomposed by means of heat.

8. Use of the compounds according to one of Claims 1 to 6, as components of liquid crystal dielectrics for electrooptical display elements.

9. Liquid crystal dielectric for electrooptical display elements with at least two liquid crystal components, characterized in that at least one component is a compound according to one of Claims 1 to 6.

10. Electrooptical display element, characterized in that it contains a dielectric according to Claim 9.

## Revendications

1. Composés de la pyrimidine contenant du fluor de formule I

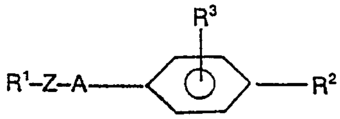

I

dans laquelle

$R^1$ et $R^2$ représentent H, R, ou OR, et l'un d'entre eux est aussi CN ou F, ou, si A est une liaison covalente, ils représentent aussi un 4-$R^4$-phényle ou un 4-R-cyclohexyle, où $R^4$ représente R, OR, CN ou F, et R est un groupe alkyle ayant de 1 à 12 atomes de C, $R^3$ représente F ou aussi H si l'un des restes $R^1$ ou $R^2$ est F ou un 4-fluorophényle,

Z est un 1,3-pyrimidine-2,5-diyle et

A est un 1,4-phénylène, un 1,4-cyclohexylène ou une liaison covalente,

ainsi que ceux de formules

| | |
|---|---|
| R–Ph–Z–PhF–CN | I dj |
| RO–Ph–Z–PhF–CN | I dk |
| R–Ph–Z–Ph–F | I dm |
| RO–Ph–Z–Ph–F | I dn |
| F–Z–Ph–Cy–R | I l |
| R–Cy–Z–Ph–F | I n |
| R–Cy–Z–PhF–F | I od |
| R–Cy–Z–PhF–CN | I oe |
| F–Z–PhF–Cy–R | I pc |
| NC–Z–PhF–Cy–R | I pd |
| R–Z–PhF–Ph–CN | I qd |
| R–Z–PhF–Ph–F | I qe |
| NC–Z–PhF–Ph–R | I qf |
| NC–Z–PhF–Ph–OR | I qg |

dans lesquelles

Ph est un 1,4-phénylène,

PhF est un 2- ou 3-fluoro-1,4-phénylène,

Cy est une 1,4-cyclohexylène,

R est un groupe alkyle ayant de 1 à 12 atomes de C, et

Z est une 1,3-pyrimidine-2,5-diyle.

2. Composés de la pyrimidine selon la revendication 1, caractérisés par les formules I a et I c

| | |
|---|---|
| $R^1$–Z–Ph–F | I a |
| $R^1$–Z–Ph–Ph–F | I c |

dans lesquelles

$R^1$ représente un alkyle à chaîne droite ayant de 2 à 8 atomes de carbone,

Z représente une 1,3-pyrimidine-2,5-diyle, et

Ph représente un 1,4-phénylène.

3. Composés de la pyrimidine selon la revendication 1, caractérisés par les formules I bc, I dj, I fd, I jd et I oe,

| | |
|---|---|
| R–Z–PhF–CN | I bc |
| R–Ph–Z–PhF–CN | I dj |
| R–Z–Ph–PhF–CN | I fd |
| R–Z–Cy–PhF–CN | I jd |
| R–Cy–Z–PhF–CN | I oe |

dans lesquelles

R représente un alkyle à chaîne droite ayant de 2 à 8 atomes de carbone,

Z représente une 1,3-pyrimidine-2,5-diyle,

Ph représente un 1,4-phénylène,

Cy représente un 1,4-cyclohexylène, et

PhF représente un 2- ou 3-fluoro-1,4-phénylène.

4. Composés de la pyrimidine selon la revendication 1, caractérisés par les formules I bd, I fe, I je et I od,

| | |
|---|---|
| R–Z–PhF–F | I bd |
| R–Z–Ph–PhF–F | I fe |
| R–Z–Cy–PhF–F | I je |
| R–Cy–Z–PhF–F | I od |

dans lesquelles

R représente un alkyle à chaîne droite ayant de 2 à 8 atomes de carbone,

Z représente une 1,3-pyrimidine-2,5-diyle,

Ph représente un 1,4-phénylène,

Cy représente un 1,4-cyclohexylène, et

PhF représente un 2- ou 3-fluoro-1,4-phénylène.

5. Dérivés de la pyrimidine selon la revendication 1, caractérisés en ce que un des restes $R^1$ et $R^2$ est un trans-4-R-cyclohexyle.

6. Dérivés de la pyrimidine selon la revendication 1, caractérisés en ce qu'un des restes $R^1$ et $R^2$ est un alkyle et l'autre est CN.

7. Procédé pour préparer des composés de la pyrimidine contenant du fluor de formule I selon la revendication 1, caractérisé en ce que

(a) on deshalogène par réduction une halogénopyrimidine de formule II

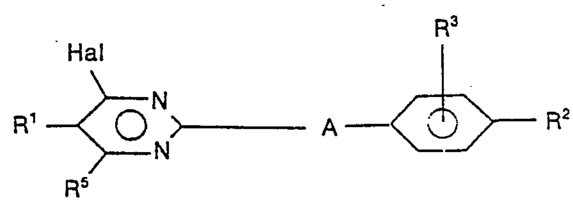

II

dans laquelle

$R^5$ représente H ou Hal, et

Hal représente Cl ou Br, et $R^1$, $R^2$, $R^3$ et A ont les significations données précedemment, ou

(b) on diazote un composé correspondant à la formule I, mais qui comporte un ou plusieurs groupements amine à la place d'un ou plusieurs atomes de fluor, on le transforme en tétrafluoroborate de diazonium correspondant, et on décompose celui-ci thermiquement.

8. Utilisation des composés selon l'une des revendications 1 à 6 comme composants de diélectriques à cristaux liquides pour des éléments indicateurs électrooptiques.

9. Diélectrique à cristaux liquides pour éléments indicateurs électrooptiques comportant au moins deux composants à cristaux liquides, caractérisé en ce que au moins un composant est un composé selon l'une des revendications 1 à 6.

10. Elément indicateur électrooptique, caractérisé en ce qu'il comporte un diélectrique selon la revendication 9.